(19) Europäisches Patentamt / European Patent Office / Office européen des brevets

(11) **EP 3 679 004 B1**

(12) **FASCICULE DE BREVET EUROPEEN**

(45) Date de publication et mention de la délivrance du brevet:
**24.01.2024   Bulletin 2024/04**

(21) Numéro de dépôt: **18759974.1**

(22) Date de dépôt: **04.09.2018**

(51) Classification Internationale des Brevets (IPC):
**C07C 7/13** *(2006.01)*       **C07C 7/14** *(2006.01)*
**C07C 15/08** *(2006.01)*

(52) Classification Coopérative des Brevets (CPC):
(C-Sets disponibles)
**C07C 7/13; C07C 7/14**                    (Cont.)

(86) Numéro de dépôt international:
**PCT/EP2018/073734**

(87) Numéro de publication internationale:
**WO 2019/043249 (07.03.2019 Gazette 2019/10)**

(54) **PROCEDE HYBRIDE DE PRODUCTION DE PARAXYLENE DE HAUTE PURETE AVEC SOLVANT TOLUENE**

HYBRIDVERFAHREN ZUR HERSTELLUNG VON HOCHREINEM PARA-XYLOL MIT TOLUOLLÖSUNGSMITTEL

HYBRID PROCESS FOR PRODUCING HIGH-PURITY PARA-XYLENE WITH TOLUENE SOLVENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **04.09.2017   FR 1758129**

(43) Date de publication de la demande:
**15.07.2020   Bulletin 2020/29**

(73) Titulaires:
• **IFP Energies nouvelles**
  **92500 Rueil-Malmaison (FR)**
• **Arkema France**
  **92700 Colombes (FR)**

(72) Inventeurs:
• **LAROCHE, Catherine**
  **92852 Rueil-Malmaison Cedex (FR)**
• **JOSSERAND, Morgane**
  **69008 Lyon (FR)**
• **RICHARD, Danielle**
  **69003 Lyon (FR)**

• **PEREZ-PELLITERO, Javier**
  **69007 Lyon (FR)**
• **BOUVIER, Ludivine**
  **64300 Orthez (FR)**

(74) Mandataire: **Bandpay & Greuter**
  **30, rue Notre-Dame des Victoires**
  **75002 Paris (FR)**

(56) Documents cités:
**EP-A1- 0 531 191      CN-A- 103 508 837
FR-A1- 2 681 066      FR-A1- 2 693 187
FR-A1- 2 925 366**

(52) Classification Coopérative des Brevets (CPC):
(Cont.)

C-Sets
**C07C 7/13, C07C 15/08;**
**C07C 7/14, C07C 15/08**

**Description**

**DOMAINE TECHNIQUE**

L'invention concerne un procédé de production de paraxylène de haute pureté à partir d'une charge d'hydrocarbures aromatiques contenant des isomères à 8 atomes de carbone. Plus précisément l'invention concerne un procédé hybride comprenant une étape d'adsorption du paraxylène et une étape de cristallisation, utilisant le toluène comme solvant commun et un adsorbant particulier à base de zéolithe faujasite contenant du baryum.

**ART ANTERIEUR**

Le marché du paraxylène de haute pureté est considéré comme un marché en forte expansion, ses débouchés étant principalement la production d'acide téréphtalique (PTA) obtenu par oxydation du paraxylène, à l'origine des fibres polyesters utilisées pour les vêtements et les résines et films de polyéthylène-téréphtalate (PET).

Le paraxylène de haute pureté est produit par valorisation des xylènes selon un procédé dit de « boucle C8-aromatiques », incluant des étapes de séparation (élimination des composés lourds dans la « colonne des xylènes », extraction du paraxylène) et d'isomérisation des xylènes. L'extraction du paraxylène de haute pureté par adsorption sélective est bien connue de l'art antérieur. L'arrière-plan technologique décrivant la production de paraxylène à très haute pureté est illustré dans le brevet FR 2 681 066 (Institut Français du Pétrole), et se base sur la séparation du paraxylène à partir d'une charge d'hydrocarbures aromatiques essentiellement à 8 atomes de carbone au sein d'un adsorbeur par contact avec un lit d'adsorbant zéolithique en présence d'un solvant de désorption (désorbant) approprié.

Pour ce type de séparation, on utilise une famille de procédés d'adsorption et de dispositifs associés connus sous le nom de procédés ou dispositifs de séparation chromatographique ou "lit mobile simulé" ou "contre-courant simulé", que nous désignerons ci-après par l'appellation de procédés et dispositifs « SMB » similaires à ceux décrits dans les brevets US 2 985 589, US 4 402 832 et US 4 498 991, et qui s'appliquent, entre autres, aux coupes aromatiques en C8.

Par ce type de procédé, on obtient un flux d'extrait contenant du solvant de désorption et essentiellement du paraxylène avec une très haute pureté, généralement supérieure à 99,5% et typiquement supérieure à 99,7%, et un flux de raffinat contenant du désorbant, du métaxylène, de l'orthoxylène, de l'éthylbenzène, et pratiquement pas de paraxylène.

Une autre méthode connue de séparation du paraxylène à partir d'une charge d'hydrocarbures aromatiques essentiellement à 8 atomes de carbone consiste à procéder à une cristallisation fractionnée. Les procédés décrits dans les brevets US 3,177,265 et 3,467,724 utilisent deux étapes de cristallisation : la première est réalisée entre -70°C et -50°C, et la seconde aux alentours de -10°C. Les inconvénients principaux de cette méthode sont un taux de récupération maximal par passe limité à environ 60% en raison de l'existence d'eutectiques entre le paraxylène et les autres isomères aromatiques en C8, et une dépense énergétique importante liée au niveau thermique de la première étape.

Le brevet FR 2 681 066 décrit un procédé hybride, couplant une première étape de séparation par chromatographie liquide de type « SMB » avec une seconde étape de cristallisation.

Les avantages d'un tel couplage par rapport à un procédé exclusivement chromatographique type SMB, résident dans le fait que le paraxylène produit au niveau de l'extrait à l'étape de séparation par chromatographie liquide est de faible pureté, typiquement 75 à 98%, ce qui permet :

- une productivité considérablement améliorée (volume de charge traitée par volume d'adsorbant et par heure),
- un plus faible ratio de débit de solvant de désorption sur débit de charge injectée,
- et l'emploi d'un nombre de lits distincts plus faible.

L'extrait produit lors de cette première étape contient du paraxylène déjà concentré qui est ensuite purifié lors de la seconde étape de cristallisation pour atteindre une très haute pureté d'au moins 99,3%, et typiquement d'au moins 99,7%. Si la teneur en paraxylène en entrée de cristallisation est comprise entre 85 et 90%, la température de cristallisation est comprise entre -5°C et -15°C. Ce niveau de température correspond typiquement au niveau de température de la seconde étape d'un procédé de séparation par cristallisation, la première opérant typiquement entre -70 et -50°C. Ainsi les avantages d'un tel couplage par rapport à un procédé exclusivement par cristallisation, sont :

- éviter des coûts opératoires prohibitifs associés à la première étape de cristallisation
- atteindre des taux de récupération (rendement) du paraxylène d'au moins 98%.

Ce couplage permet ainsi à la fois des gains CAPEX (coûts d'investissement) sur la section chromatographique grâce à un nombre de lits réduit, et une productivité élevée, ainsi que des gains OPEX (coûts opératoires, c'est-à-dire les coûts d'utilités) via un taux de désorbant réduit sur la section chromatographique et une section de cristallisation limitant les frigories.

Le brevet CN103508837 propose également un procédé hybride de ce type, couplant une étape de séparation chromatographique type SMB et une étape de cristallisation. Dans le procédé décrit, le solvant de lavage des cristaux est le toluène, tandis que le solvant de désorption est le diéthylbenzène.

Le brevet FR 2 681 066 décrit que le solvant de désorption et le solvant de lavage peuvent être un solvant de point d'ébullition inférieur à celui de la charge tel que le toluène, ou supérieur à celui de la charge tel que le cumène, mais également que le solvant de désorption peut être un solvant de point d'ébullition supérieur à celui de la charge tel que le paradiéthylbenzène et le solvant de lavage peut être un solvant de point d'ébullition inférieur à celui de la charge tel que le toluène.

Le brevet FR 2 681 066 montre également que la sélection adéquate du solvant de lavage et du solvant de désorption permet de créer une synergie entre les étapes de chromatographie et de cristallisation quand ledit solvant est commun aux deux étapes, en éliminant une unité de distillation. Le toluène a été décrit comme solvant de désorption pour la séparation des xylènes avec des adsorbants constitués principalement de zéolithes X ou Y dont les sites échangeables sont occupés par des cations alcalins ou alcalino-terreux. Par ailleurs, dans les procédés de cristallisation fractionnée, il est connu d'employer le toluène comme solvant de lavage des cristaux de paraxylène.

Afin d'obtenir de bonnes performances dans un procédé hybride couplant adsorption et cristallisation, il est nécessaire de sélectionner un adsorbant efficace pour la séparation par chromatographie liquide du paraxylène en présence d'un solvant de désorption approprié, permettant de faire fonctionner l'étape de cristallisation de manière optimale afin d'obtenir du paraxylène de haute pureté tout en minimisant les coûts.

En particulier, pour obtenir de bonnes performances lors de l'étape d'adsorption, qui conditionnent l'efficacité de l'étape de cristallisation subséquente, l'adsorbant à utiliser pour la séparation chromatographique doit être efficace pour adsorber sélectivement le paraxylène dans un mélange d'hydrocarbures aromatiques, et doit également être efficacement régénéré par le solvant de désorption. Plus précisément, si le solvant commun choisi est le toluène, l'adsorbant doit notamment être capable de séparer efficacement le paraxylène des autres isomères en C8, avec notamment une sélectivité entre le paraxylène et le métaxylène élevée (par exemple d'au moins 3), et être efficacement régénéré par le toluène comme solvant de désorption.

Le brevet FR2 693 187 décrit un procédé permettant de séparer en continu du paraxylène à partir d'une charge d'hydrocarbures en $C_8$ aromatique, le procédé comprenant une étape de séparation de paraxylène à basse pureté par chromatographie d'adsorption à co-courant simulé; une étape de purification et de lavage du paraxylène basse pureté au moyen de l'étage de recristallisation; et une étape d'isomérisation catalytique. avantageusement le toluène comme solvant de désorption et comme solvant de lavage. Dans le procédé décrit, le toluène peut être utilisé comme solvant de désorption et solvant de lavage.

Le brevet EP0 531 191 décrit un procédé et un dispositif permettant de séparer en continu du haute pureté à partir d'une charge d'hydrocarbures en $C_8$ aromatique, le procédé comprenant une étape de séparation de paraxylène à basse pureté par chromatographie d'adsorption à lit mobile simulé; une étape de purification et de lavage du paraxylène basse pureté au moyen de l'étage de recristallisation; et une étape d'isomérisation catalytique. Dans le procédé décrit, le solvant de désorption et de lavage peut être le toluène.

Il est connu dans l'art antérieur que des adsorbants zéolithiques comprenant au moins de la zéolithe Faujasite (FAU) de type X ou Y et comprenant, outre des cations sodium, des ions baryum, potassium ou strontium, seuls ou en mélanges, sont efficaces pour adsorber sélectivement le paraxylène dans un mélange d'hydrocarbures aromatiques (US 3 558 730, US 3 558 732, US 3 663 638 et US 3 878 127).

Le brevet FR 2 681 066 précise que lorsque le solvant de désorption est le toluène, la zéolithe préférée est la zéolithe Y échangée à la fois au baryum et au potassium, alors que lorsque le solvant de désorption est le paradiéthylbenzène (PDEB), la zéolithe préférée est la zéolithe X échangée quasi totalement au baryum.

Cependant la zéolithe Y échangée à la fois au baryum et au potassium, dont l'utilisation est préconisée dans le brevet FR 2 681 066 dans un SMB en présence de toluène comme solvant de désorption, ne permet pas d'obtenir une sélectivité entre le paraxylène et le métaxylène satisfaisante. En effet, la sélectivité obtenue avec cette zéolithe entre le paraxylène et le métaxylène est de l'ordre de 2,5.

De façon surprenante, les adsorbants contenant majoritairement de la zéolite FAU, contenant du baryum et de paramètre de maille « a » supérieur à 25,100 Å, (soit 2,5100 nm) telle que la teneur en baryum de l'adsorbant, exprimée en poids d'oxyde de baryum BaO, par rapport au poids total de l'adsorbant, soit comprise entre 30% et 41%, mis en oeuvre dans un procédé hybride comprenant une étape d'adsorption et une étape de cristallisation, en présence de toluène à la fois comme solvant de désorption et comme solvant de lavage permettent de mettre en oeuvre une quantité plus faible de désorbant pour régénérer l'adsorbant, tout en obtenant une sélectivité ($\alpha$) pour le paraxylène par rapport au métaxylène (PX/MX) et par rapport à l'éthylbenzène (PX/EB) élevée (notamment une sélectivité PX/MX > 3 et une sélectivité PX/EB > 2.4).

Ledit paramètre de maille « a » est mesuré sur l'adsorbant final, après échange avec du baryum.

L'invention n'exclut pas la présence d'autres cations alcalins ou alcalino-terreux, en faible quantité, ou à l'état de traces, typiquement à une teneur inférieure à 5% en poids d'oxyde par rapport au poids total de l'adsorbant.

**Résumé de l'invention**

L'invention concerne un procédé hybride de production de para-xylène de haute pureté à partir d'une charge de coupes d'isomères d'hydrocarbures aromatiques contenant 8 atomes de carbone, en phase liquide, comprenant :

a) une étape de séparation du para-xylène par adsorption à contre-courant simulé au moyen d'un adsorbant zéolithique et d'un solvant de désorption, ledit adsorbant comprenant :

- au moins une zéolithe majoritaire de type faujasite de paramètre de maille « a » supérieur à 25,100 Å
- du baryum telle que la teneur en oxyde de baryum BaO soit comprise entre 30% et 41% poids bornes incluses, par rapport au poids total de l'adsorbant,

pour obtenir un flux de paraxylène purifié ;

b) une étape de cristallisation du paraxylène à partir du flux purifié de paraxylène issu de l'étape de séparation, à une température comprise entre 0 et -25°C, suivie d'un lavage des cristaux avec un solvant de lavage, pour obtenir le paraxylène de haute pureté,

le solvant de désorption dans l'étape de séparation et le solvant de lavage dans l'étape de cristallisation étant le toluène, l'adsorbant comprenant une zéolithe LSX ou un mélange de zéolithes de type faujasite, ce mélange étant une zéolithe X et une zéolithe LSX, la zéolithe LSX étant majoritaire. Dans un mode de réalisation, la surface externe dudit adsorbant zéolithique, mesurée par adsorption d'azote, est inférieure à 100 $m^2.g^{-1}$.

De préférence, la teneur en oxyde de baryum est comprise entre 33 et 41% bornes incluses, de manière encore plus préférée entre 33 et 38% bornes incluses, par rapport au poids total de l'adsorbant.

La température de l'étape de cristallisation est avantageusement comprise entre -5°C et -15°C.

Le taux de lavage des cristaux est compris entre 0,8 à 2 volumes de toluène par volume de cristaux de paraxylène.

Le nombre de lits dans l'étape de séparation par adsorption est avantageusement compris entre 4 et 24, de préférence entre 8 et 12.

Dans un mode de réalisation du procédé selon l'invention, l'étape de séparation par adsorption opère avec un nombre de lits compris entre 4 et 24, un nombre de zones au moins égal à 4, une température comprise entre 100°C et 250°C, une pression comprise entre la pression de bulle du toluène à la température du procédé et 3 MPa, un temps de cycle, correspondant au temps entre deux injections de désorbant sur un lit donné, compris entre 4 et 18 min, un rapport des débits de désorbant par rapport à la charge de 0,7 à 2,5, un taux de recyclage (rapport du débit moyen des zones pondéré du nombre de lits présents dans chaque zone sur le débit de charge) compris entre 2 et 12.

De préférence, l'étape de séparation par adsorption opère à une température comprise entre 150°C et 180°C.

De préférence, l'étape de séparation par adsorption opère avec un taux de recyclage (rapport du débit moyen des zones pondéré du nombre de lits présents dans chaque zone sur le débit de charge) compris entre 2,5 et 4.

L'adsorbant utilisé dans le procédé selon l'invention peut être obtenu par toute méthode connue de l'homme du métier, et en particulier selon l'une des méthodes décrites dans les demandes de brevets EP2237878, EP2237877, et WO2016/075281.

De manière avantageuse, l'adsorbant est préparé selon les étapes suivantes :

a) mélange des cristaux de zéolithe faujasite en poudre de granulométrie souhaitée, en présence d'eau avec au moins un liant à base d'une argile ou d'un mélange d'argiles, contenant au moins 80 % en poids d'argile zéolithisable et éventuellement une source de silice ;

b) mise en forme du mélange obtenu en a) pour produire des agglomérés, puis séchage, éventuellement suivi d'une étape de tamisage et/ou de cyclonage ;

c) calcination des agglomérés obtenus en b) à une température allant préférentiellement de 500°C à 600°C ;

d) éventuelle zéolithisation du liant par mise en contact des agglomérés calcinés issus de l'étape c) avec une solution aqueuse basique alcaline suivie d'un lavage ;

e) échange ionique des agglomérés zéolithiques à base de zéolithe LSX, ou de X et LSX obtenus en c) ou en d) par des ions baryum, suivi d'un lavage et d'un séchage du produit ainsi traité ;

f) activation thermique des agglomérés échangés de l'étape e), par exemple à une température allant de 200 à 300°C.

## Description détaillée de l'invention

Dans la suite de la description, on entend par zéolithe X une zéolithe de type faujasite ayant un rapport atomique Si/Al compris entre 1,15, borne exclue et 1,5, borne comprise.

On entend par zéolithe LSX (Low Silica X) une zéolithe de type faujasite à faible teneur en silice ayant un rapport atomique Si/Al proche de 1, plus précisément compris entre 1 et 1,15, bornes comprises.

## Adsorbants

Les adsorbants utilisés dans le procédé hybride selon l'invention contiennent au moins une zéolithe FAU dont le paramètre de maille « a »(maille cubique telle que a=b=c) déterminé par DRX est strictement supérieur à 25,100 Å.

Il peut y avoir une ou plusieurs autres zéolithes FAU, mais dans ce cas, la ou les zéolithes FAU de paramètre de maille strictement supérieur à 25,100 Å sont majoritaires. Par zéolithe(s) majoritaire(s), on entend les zéolithes représentant une proportion en poids d'au moins 50% par rapport à l'ensemble de zéolithes FAU. Lorsque plusieurs zéolithes FAU sont présentes dans l'adsorbant, la détermination de la zéolithe majoritaire de type FAU est réalisée sur le signal DRX correspondant à 35° $\pm$ 0,6° 2 $\theta$. Lors de la présence de plusieurs zéolithes FAU, le signal en question se caractérise par un pic dédoublé, le pic de gauche correspondant à la zéolithe de paramètre de maille le plus élevé. On entend alors par zéolithe majoritaire la zéolithe dont la contribution en termes d'aire est supérieure strictement à 50 % de l'aire totale du pic dédoublé, de préférence strictement supérieure à 51%, de préférence encore strictement supérieure à 55%, et typiquement strictement supérieure à 60%.

La mesure de la contribution en termes d'aire est effectuée sur l'adsorbant final après échange au baryum. Si nécessaire et afin d'améliorer la précision de la détermination de la contribution de chaque zéolithe, une méthode de déconvolution de pics (disponible dans les outils d'analyse des pics de DRX) peut s'appliquer. Dans un mode de réalisation particulièrement préféré, la proportion de zéolithes autres que la FAU est toujours inférieure à 10 %.

L'invention n'exclut pas la présence d'autres cations alcalins ou alcalino-terreux, en faible quantité, ou à l'état de traces, typiquement à une teneur inférieure à 5% en poids d'oxyde par rapport au poids total de l'adsorbant.

Les adsorbants préférés dans le cadre de l'invention sont les adsorbants comprenant une zéolithe LSX à porosité hiérarchisée ou de haute surface externe, c'est-à-dire une surface externe de l'adsorbant comprise entre 20 et 100 m$^2$/g,

Les adsorbants zéolithiques utilisés dans la présente invention peuvent être obtenus selon toute technique connue de l'homme du métier, et en particulier selon l'une des méthodes décrites dans les demandes de brevets EP2237878, EP2237877, et WO2016/075281.

Les adsorbants zéolithiques utilisés dans la présente invention sont notamment obtenus avantageusement selon un procédé de préparation tel que décrit précédemment:

a) mélange des cristaux de zéolithe faujasite, par exemple des cristaux de zéolithe LSX, ou des cristaux de zéolithe X et LSX, en poudre de granulométrie souhaitée, en présence d'eau avec au moins un liant à base d'une argile ou d'un mélange d'argiles, contenant au moins 80% de préférence, au moins 90%, de préférence encore au moins 95% en poids d'argile zéolithisable et éventuellement une source de silice ;

b) mise en forme du mélange obtenu en a) pour produire des agglomérés, puis séchage, éventuellement suivi d'une étape de tamisage et/ou de cyclonage ;

c) calcination des agglomérés obtenus en b) à une température allant préférentiellement de 500°C à 600°C ;

d) éventuelle zéolithisation du liant par mise en contact des agglomérés calcinés issus de l'étape c) avec une solution aqueuse basique alcaline suivie d'un lavage ;

e) échange ionique des agglomérés zéolithiques à base de zéolithe LSX, ou de X et LSX obtenus en c) ou en d) par des ions baryum, suivi d'un lavage et d'un séchage du produit ainsi traité ;

f) activation thermique du produit issu de l'étape e), préférentiellement à une température allant de 200 à 300°C.

L'étape b) de mise en forme permet d'obtenir des agglomérés zéolithiques présentant une résistance mécanique suffisante pour leur utilisation dans un procédé de séparation des xylènes en lit mobile simulé. Cependant la présence de liant réduit la proportion de matière active au sens de l'adsorption (zéolithes LSX, et X).

L'étape optionnelle d) de zéolithisation du liant permet ainsi de transformer tout ou partie du liant en matière active au sens de l'adsorption (zéolithes LSX et X) afin d'obtenir des agglomérés sans liant, c'est à dire ne comportant plus de phase non zéolithique (en quantité typiquement inférieure à 2%), ou des agglomérés à faible teneur en liant, c'est-à-dire comportant peu (quantité typiquement comprise entre 2 et 5%) de phase non zéolithique (généralement du liant résiduel non zéolithisé ou toute autre phase amorphe après zéolithisation) dans l'aggloméré final, et ce, tout en maintenant la résistance mécanique. Le taux de phase non zéolithique (c'est-à-dire le liant résiduel non zéolithisé qui constitue une phase amorphe inerte au sens de l'adsorption, après zéolithisation) dans l'aggloméré final peut être quantifié par référence à un adsorbant composé uniquement de zéolithe, sous forme de poudre, à partir de mesures d'adsorption ou à partir d'intensités de pics de diffraction X (DRX). La zéolithe de référence choisie pour la quantification par diffraction X (DRX) est la zéolithe utilisée à l'étape a) du procédé d'obtention de l'adsorbant, et ayant subi le même échange ionique.

Les cristaux de zéolithes LSX, ou X issus de la zéolithisation du liant (transformation du liant en zéolithe) sont généralement de plus petit diamètre que les cristaux initiaux. Par conséquent, dans l'aggloméré final, les cristaux dont le diamètre moyen est inférieur ou égal à 7 $\mu$m, de préférence compris entre 0,1 $\mu$m et 4 $\mu$m, et de manière préférée entre 0,1 $\mu$m et 3 $\mu$m, présentent de manière classique une distribution granulométrique monomodale, mais on ne sortirait pas du cadre de l'invention si la distribution des diamètres des cristaux était multimodale, et en particulier bimodale, du fait de la présence de la population des cristaux issus de la zéolithisation du liant.

La mesure du paramètre de maille « a », effectuée sur l'adsorbant final après échange au baryum, permet d'identifier la ou les zéolithe(s) présente(s) dans l'adsorbant.

**Procédé hybride de production de paraxylène**

Etape de séparation par adsorption

L'invention concerne un procédé de production de paraxylène de haute pureté à partir d'une charge d'hydrocarbures aromatiques contenant des isomères à 8 atomes de carbone au moyen d'une étape d'adsorption A), suivie d'une étape de cristallisation B), chacune des deux étapes mettant en oeuvre le même solvant toluène, l'étape d'adsorption comprenant les étapes suivantes :

A1) mise en contact, dans des conditions d'adsorption adéquates, de la charge avec un lit d'adsorbant tel que décrit précédemment, de manière à adsorber sélectivement le paraxylène ;

A2) mise en contact dans des conditions de désorption, du lit d'adsorbant avec un désorbant, ou solvant de désorption, qui est le toluène ;

A3) soutirage du lit d'adsorbant d'un flux de raffinat contenant le désorbant et les produits de la charge les moins sélectivement adsorbés,

A4) soutirage du lit d'adsorbant d'un flux d'extrait contenant le désorbant et le paraxylène,

A5) séparation du flux de raffinat issu de l'étape A3 en un premier flux contenant le désorbant et un second flux contenant les produits de la charge les moins sélectivement adsorbés, et

A6) une séparation du flux d'extrait issu de l'étape A4 en un premier flux contenant le désorbant et un second flux contenant du paraxylène à un niveau de pureté supérieure ou égale à 75%, de préférence supérieure à 85%.

La performance des adsorbants zéolithiques, tels que décrits précédemment, pour le procédé de séparation du paraxylène en terme de pureté de paraxylène dans l'extrait et de rendement du procédé, est également influencée par différents paramètres du procédé, à savoir les conditions opératoires, la composition de la charge d'alimentation, la teneur en eau et le type de désorbant.

Le solvant de désorption, également appelé désorbant, utilisé dans le procédé selon l'invention est le toluène, ou plus précisément un solvant constitué majoritairement de toluène, c'est-à-dire constitué de toluène et d'éventuelles traces d'autres composants en C8.

L'étape de séparation par adsorption du para-xylène à contre-courant simulé fonctionne selon l'état de la technique qui est bien connu, et comporte au moins quatre zones, et éventuellement cinq ou six, chacune de ces zones étant constituée par un certain nombre de lits successifs, et chaque zone étant définie par sa position comprise entre un point d'alimentation et un point de soutirage. Typiquement, une unité de séparation par adsorption du paraxylène à contre-courant simulé est alimentée par au moins une charge à fractionner (contenant le paraxylène et les autres isomères en C8 aromatique) et un désorbant, parfois appelé éluant ou solvant de désorption, et l'on soutire de ladite unité au moins un raffinat contenant les isomères du paraxylène et du désorbant et un extrait contenant le paraxylène et du désorbant.

Les conditions opératoires de l'unité industrielle d'adsorption à contre-courant simulé mettant en oeuvre les adsorbants zéolithiques tels que décrits précédemment, sont typiquement les suivantes :

- nombre de lits compris entre 4 et 24, de préférence entre 8 et 12 ;

- nombre de zones : au moins 4 ;

- température 100°C à 250°C, de préférence 150°C à 180°C ;

- pression comprise entre la pression de bulle du toluène à la température du procédé et 3 MPa ;

- temps de cycle, correspondant au temps entre deux injections de désorbant sur un lit donné, compris entre 4 et 18 min ;

- rapport des débits désorbant sur charge 0,7 à 2,5 ;

- taux de recyclage (rapport du débit moyen des zones pondéré du nombre de lits présents dans chaque zone sur le débit de charge) de 2 à 12, de préférence 2,5 à 4.

La teneur en eau dans les effluents hydrocarbonés est préférentiellement ajustée entre 20 ppm et 150 ppm pour une température du procédé de 150°C à 180°C, afin d'obtenir des résultats optimaux en productivité.

Etape de cristallisation :

L'étape de cristallisation est opérée avantageusement dans un cristalliseur et consiste en la cristallisation du paraxylène issu du flux purifié de l'étape d'adsorption, contenant du paraxylène à un niveau de pureté supérieure ou égale à 75% et allant jusqu'à 98 %. L'étape de cristallisation permet d'obtenir d'une part des cristaux de paraxylène imbibés de leur liqueur-mère, et d'autre part une liqueur-mère qui peut être en partie, voire en totalité, recyclée en mélange avec la charge fraîche à l'entrée de l'unité d'adsorption en lit mobile simulé.

L'étape de cristallisation comprend également un lavage des cristaux issus du cristalliseur, à l'issue duquel on récupère du paraxylène à une pureté d'au moins 99,3%, de manière préférée d'au moins 99,7% et de manière très préférée d'au moins 99,8%.

Pour un flux purifié issu de l'adsorbeur et entrant dans l'étape de cristallisation, contenant de préférence de 85 à 90% de paraxylène, la température du cristalliseur est comprise entre 0 et -25°C, de préférence entre -5°C et -15°C et le taux de lavage lors du lavage des cristaux est généralement compris entre 0,8 à 2 volumes de toluène par volume de cristaux de paraxylène.

Le solvant de lavage des cristaux, utilisé dans le procédé selon l'invention est identique au solvant utilisé comme désorbant dans l'unité d'adsorption, à savoir le toluène ou un solvant constitué majoritairement de toluène, c'est-à-dire constitué de toluène et d'éventuelles traces d'autres composants en C8, notamment des traces de métaxylène.

Les flux de désorbant issus des étapes A5) et A6) décrites ci-dessus sont avantageusement utilisés dans l'étape de cristallisation pour effectuer le lavage des cristaux. Le toluène impur issu du cristalliseur est envoyé à l'étape A5) décrite ci-dessus, au même titre que le flux de raffinat de l'étape d'adsorption. Un appoint de toluène comme solvant de lavage est également possible.

De manière préférée, une combinaison avantageuse selon l'invention pour produire du paraxylène à haute pureté tout en réduisant les coûts, et notamment les coûts opératoires, consiste à coupler une étape de séparation par chromatographique liquide type SMB dans un adsorbeur unique contenant un nombre de lits réduits, typiquement entre 8 et 12, avec une étape de cristallisation opérant à une température comprise entre -5°C et -15°C, en utilisant le toluène à la fois comme solvant de désorption dans le SMB et comme solvant de lavage lors de l'étape de cristallisation.

**Technique de caractérisation des zéolithes présentes et de leurs paramètres de maille**

La mesure du paramètre de maille « a » permet d'identifier la ou les zéolithe(s) présente(s) dans l'adsorbant.

Les zéolithes présentes dans l'adsorbant et leur paramètre de maille sont caractérisés par analyse par diffraction de rayons X, connue de l'homme du métier sous l'acronyme DRX. Cette analyse est réalisée sur un appareil de la marque Panalytical, sur un échantillon d'adsorbant zéolithique préalablement broyé et tamisé (fraction inférieure à 50µm). L'échantillon est saturé en eau de façon à être stable pendant la durée de l'analyse. Pour ce faire, l'échantillon est séché à 110°C dans une étuve, puis placé, en couche mince, dans une enceinte fermée à une humidité relative de 55% à température ambiante, pendant un minimum de 48 heures.

L'analyse est réalisée en ajoutant un étalon interne (5% de silicium certifié en paramètre de maille), sur une plage angulaire (en°2θ) comprise entre 5 à 72° 2 θ, avec un pas angulaire (en °) de 0,02°. Les données sont traitées avec le logiciel d'affinement TOPAS, pour obtenir une mesure du paramètre de maille des zéolithes présentes dans l'adsorbant avec précision (à +/- 0,005 Å).

Dans le cas d'un système cristallin cubique, comme le cas de la zéolithe de structure FAU, les dimensions de la maille cristalline dans les 3 directions de l'espace sont identiques et donc définies par une seule valeur du paramètre de maille « a ». Dans le logiciel TOPAS, les pics du diffractogramme seront modélisés par une fonction de type Pseudo-Voigt, et le fond par une fonction polynomiale.

Dans le cas où plusieurs zéolithes FAU sont présentes dans l'adsorbant, ce fait est détecté par DRX, et visible sur le diffractogramme par la présence d'un dédoublement de pic, particulièrement visible dans la plage angulaire (en°2θ) comprise entre 34 à 45° 2θ. Dans le cas de mélanges de zéolithes FAU, le pic dédoublé sélectionné pour définir la zéolithe majoritaire dans l'adsorbant est le pic correspondant à 35° +/- 0,6° 2θ, le pic de gauche correspondant au pic de la zéolithe de paramètre de maille le plus élevé (c'est-à-dire la LSX de paramètre de maille compris entre 25,18 et 25,23 Å dans les adsorbants des exemples). On entend par zéolithe majoritaire dans l'adsorbant, la zéolithe dont la contribution en termes d'aire à l'intensité totale du pic dédoublé est supérieure ou égale à 50 %. Si nécessaire et afin d'améliorer la précision de la détermination de la contribution de chaque zéolithe, une méthode de déconvolution de pics peut s'appliquer.

La surface externe de l'adsorbant est usuellement déterminée à partir de la mesure de l'isotherme d'adsorption de gaz, tel que l'azote, à sa température de liquéfaction.

Préalablement à l'adsorption, l'adsorbant zéolithique est dégazé entre 300°C et 450°C pendant une durée comprise entre 9 heures et 16 heures, sous vide (P < 6,7.10-4 Pa). La mesure de l'isotherme d'adsorption d'azote à 77K est ensuite effectuée sur un appareil de type ASAP 2020 M de Micromeritics, en prenant au moins 35 points de mesure à des pressions relatives de rapport P/P0 comprise entre 0,002 et 1.

La surface externe est déterminée par la méthode du t-plot à partir de l'isotherme obtenue, en appliquant la norme ISO 15901-3:2007 et en calculant l'épaisseur statistique t par l'équation de Harkins-Jura. La surface externe est obtenue par régression linéaire sur les points du t-plot compris entre 0,45 nm et 0,57 nm, à partir de la pente de la régression linéaire.

La surface externe s'exprime en $m^2$ par gramme d'adsorbant anhydre.

## Liste des figures

**Figure 1A :** Diffractogramme obtenu par diffraction de rayons X sur les adsorbants C (comparatif) et F (conforme à l'invention) et zoom sur le pic dédoublé à 35°

**Figure 1 B:** Diffractogramme obtenu par diffraction de rayons X sur les adsorbants C (comparatif) et F (conforme à l'invention) et zoom sur le pic dédoublé à 35°, sous forme décalée

**Figure 2 :** Diffractogramme obtenu par diffraction de rayons X sur l'adsorbant G (conforme à l'invention)
En annexe, figure le tableau 4 listant les pics et leur intensité pour chacun des diffractogrammes des figures 1A, 1B et 2.

**Figures 3A et 3B :** Courbes de perçage de chaque composé présent dans l'effluent prélevé à la sortie de la colonne chromatographique représentant l'évolution des concentrations de chaque composé en fonction du volume élué, lors du test de perçage (étape d'injection de la charge) et lors du test de déperçage (injection du désorbant), dans le cas de l'adsorbant A (BaX).

**Figure 4 :** Représentation graphique du ratio $R_{des/charge}$ des différents adsorbants testés aux exemples 1 et 2 en fonction du paramètre de maille de la zéolithe majoritaire.

**EXEMPLES**

**Exemple 1 (comparatif)** : Test de séparation en colonne chromatographique avec adsorbants selon l'art antérieur

On réalise des tests de séparation en utilisant une colonne chromatographique dans laquelle est conditionné le solide zéolithique.

Les solides testés sont :

- A : Adsorbant préparé selon l'exemple 4 du brevet FR2903978, à base de zéolithe X échangée au baryum, de paramètre de maille mesuré 25,025 Å.
- B : Adsorbant à base de zéolithe Y échangée à la fois au baryum (45 à 65% de sites) et au potassium (35 à 55% des sites) tel que décrit dans le brevet FR2681066 et tel que défini dans le brevet US3558730, de paramètre de maille mesuré 24,712 Å.
- C : Adsorbant à base d'un mélange X - LSX, majoritaire en X, préparé avec un ratio molaire de cristaux LSX/X égal à 0,54, échangé au baryum selon l'exemple 5 du FR 2925367 de paramètre de maille mesuré 25,034 Å pour la zéolithe X et 25,226 Å pour la zéolithe LSX. La zéolithe majoritaire est donc la zéolithe X de paramètre de maille inférieur à 25,100 Å.
- La température des tests est de 163°C correspondant à la température couramment préconisée pour la séparation du paraxylène en procédé hybride couplant adsorption et cristallisation.

Les tests réalisés sont des tests de type perçage/déperçage avec une charge contenant 10%volume d'iso-octane (iC8) utilisé comme traceur, 45%volume de paraxylène (PX) et 45%volume de métaxylène (MX) et avec un solvant de désorption (désorbant) qui est le toluène.

- Le test de perçage correspond à l'étape d'injection de la charge dans la colonne initialement saturée en désorbant.
- Le test de déperçage correspond à l'étape d'injection du désorbant, et est réalisée à la fin de l'étape précédente d'injection de charge.

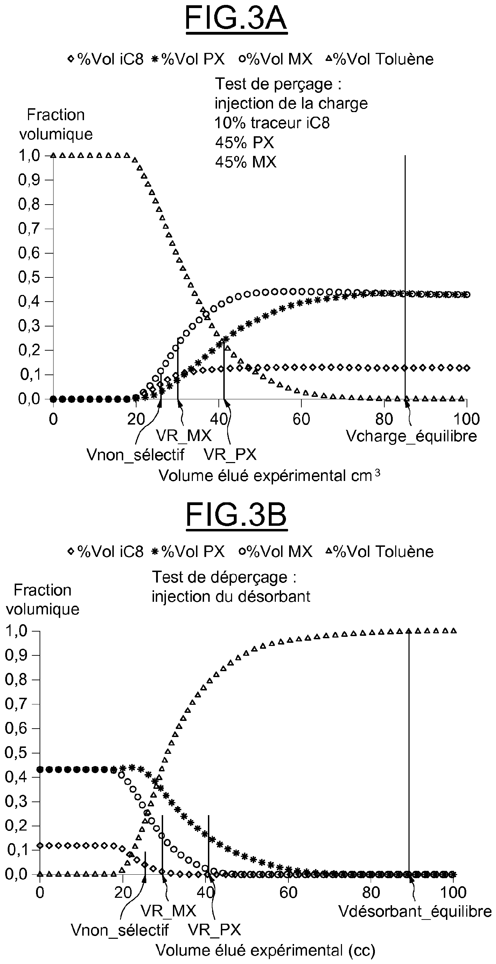

Les courbes représentées sur les figures 3A et 3B correspondent aux courbes de perçage de chaque composé présent dans l'effluent prélevé à la sortie de la colonne chromatographique : elles représentent l'évolution des concentrations de chaque composé en fonction du volume élué, lors du test de perçage (étape d'injection de la charge) et lors du test de déperçage (injection du désorbant), dans le cas de l'adsorbant A (BaX).

L'étape d'injection de la charge est terminée lorsque la concentration en désorbant de l'effluent prélevé en sortie de colonne chromatographique devient nulle, c'est-à-dire lorsque la composition de l'effluent prélevé est identique à la composition de la charge injectée : le volume de charge injecté à ce point est noté Vcharge_équilibre et traduit la quantité de charge à injecter nécessaire pour saturer complètement les volumes poreux au sein de la colonne, à savoir l'ensemble des volumes non sélectifs (volume inter-grain et volume macro et mésoporeux intra-grain) et du volume sélectif (volume microporeux de l'adsorbant).

L'iso-octane (iC8) présent à 10% en volume dans la charge est utilisé comme traceur des volumes non-sélectifs (volume inter-grain et volume macro et mésoporeux intra-grain) : la mesure du premier moment de la courbe de perçage de l'iC8 donne ainsi l'estimation du volume non-sélectif, noté Vnon_sélectif.

Ainsi en retranchant ce volume non sélectif au volume d'injection de charge à l'équilibre Vcharge_équilibre, on obtient le volume de charge à injecter nécessaire pour saturer le volume microporeux $V_\mu$ de l'adsorbant, à savoir (Vcharge_équilibre - Vnon_sélectif).

Ainsi en multipliant le volume (Vcharge_équilibre - Vnon_sélectif) par la fraction $x_{PX}$ de PX contenu dans la charge, on obtient le volume de paraxylène PX à injecter lors du test de perçage pour atteindre l'équilibre d'adsorption dans le volume microporeux.

La sélectivité $\alpha_{PX/MX}$ est le ratio des quantités adsorbées de PX (paraxylène) et de MX (métaxylène) divisé par le ratio des concentrations de PX et MX dans la charge $x_{PX}$ et $x_{MX}$. Les quantités adsorbées de PX et de MX sont obtenues à partir des premiers moments des courbes du PX et du MX, notés $V_{R\_PX}$ et $V_{R\_MX}$, et du premier moment de la courbe du traceur donnant le volume non sélectif noté Vnon_sélectif. Ainsi la sélectivité $\alpha_{PX/MX}$ est calculée par l'équation suivante :

$$\alpha_{PX/MX} = \frac{\left(V_{R\_PX} - V_{non\_sélectif}\right) x_{MX}}{\left(V_{R\_PX} - V_{non\_sélectif}\right) x_{PX}}$$

De manière analogue, l'étape d'injection du désorbant est terminée lorsque la concentration en désorbant de l'effluent prélevé en sortie de colonne chromatographique devient 100% : le volume de désorbant injecté à ce stade est noté Vdésorbant_équilibre et traduit la quantité de désorbant à injecter nécessaire pour régénérer complètement les volumes poreux au sein de la colonne, à savoir l'ensemble des volumes non sélectifs (volume inter-grain et volume macro et mésoporeux intra-grain) et du volume sélectif (volume microporeux de l'adsorbant).

La mesure du premier moment de la courbe de déperçage de l'iC8 donne l'estimation du volume non-sélectif, noté Vnon_sélectif.

En retranchant ce volume non sélectif au volume d'injection de désorbant à l'équilibre Vdésorbant_équilibre, on obtient le volume de désorbant à injecter nécessaire pour régénérer complètement le volume microporeux de l'adsorbant, à savoir (Vdésorbant_équilibre - Vnon_sélectif).

Les figures 3A et 3B montrent, que pour l'adsorbant comparatif A, le volume de désorbant à l'équilibre est supérieur au volume de charge à l'équilibre.

A partir des tests de perçage/déperçage, le ratio $R_{Des/Charge}$ calculé par :

$$R_{Des/Charge} = (Vdésorbant\_équilibre - Vnon\_sélectif) / (Vcharge\_équilibre - Vnon\_sélectif)$$

doit représenter un processus similaire au ratio D/F utilisé lors de la séparation du paraxylène en lit mobile simulé, c'est-à-dire le ratio entre le débit de désorbant et le débit de charge. Ce paramètre traduit en effet la quantité de désorbant nécessaire pour désorber complètement les composés de la charge préalablement adsorbés dans le volume microporeux de l'adsorbant.

Les ratios volumiques $R_{Des/Charge}$ calculés à partir des courbes de concentration des différents composés pour les différents adsorbants testés sont donnés dans le tableau 1 suivant :

Tableau 1 : Résultats des tests de perçage sur les adsorbants A, B, C en termes de sélectivité PX/MX et de ratio volumique désorbant/charge

|  | Si/Al du produit fini | Teneur en BaO (%poids) | Paramètre de maille mesuré Å | Comparaison des pics de zéolithe de paramètre de maille > 25, 100 Å et < 25,100 Å | $\alpha_{PX/MX}$ | $R_{Des/Charge}$ |
|---|---|---|---|---|---|---|
| **Comparatif** |  |  |  |  |  |  |
| A | 1,25 | 35,2 | 25,025 |  | 3,33 | 1,13 |
| B | 2,3 | 10,8 | 24,712 |  | 2,55 | 1,29 |
| C | 1,16 | 36,7 | 25,034 (X) et 25,226 (LSX) | Aire Pic X > Aire Pic LSX | 3,65 | 1,03 |

Exemple 2 (selon l'invention)

Des tests tels que décrits dans l'exemple 1 sont réalisés avec des solides zéolithiques selon l'invention.

- D : Adsorbant préparé selon l'exemple 6 du brevet FR2925366, à base de zéolithe LSX échangée au baryum, de paramètre de maille mesuré 25,200 Å.

- E : Adsorbant comprenant au moins une zéolithe de structure FAU de type LSX, comprenant du baryum, de surface externe mesurée par adsorption d'azote égale à 64 $m^2.g^{-1}$, préparé selon l'exemple 3 de la demande de brevet FR3028430 de paramètre de maille mesuré 25,204 Å.

- F : Adsorbant à base d'un mélange de zéolithes X - LSX (35% - 65%), majoritaire en LSX, préparé avec un ratio molaire de cristaux LSX/X égal à 1,86 et échangé au baryum selon l'exemple 5 du brevet FR2925367, de paramètre de maille mesuré 25,031 Å (X) et 25,223 Å (LSX)

- G : Adsorbant à base d'un mélange de zéolithes X - LSX majoritaire en LSX, préparé avec un ratio molaire de cristaux LSX/X égal à 1, zéolithisé et échangé au baryum selon l'exemple 7 du brevet FR2925367, de paramètre de maille mesuré 25,003 Å (X) et 25,181 Å (LSX).

Tableau 2 : Résultats des tests de perçage sur les adsorbants D, E, F, G en termes de sélectivité PX/MX et de ratio volumique désorbant/charge

| | Si/Al du produit fini | Teneur en BaO (%poids) | Paramètre de maille mesuré Å | Comparaison des pics de zéolithe de paramètre de maille > 25,100 Å et < 25,100 Å | $\alpha_{PX/MX}$ | $R_{Des/Charge}$ |
|---|---|---|---|---|---|---|
| **Selon invention** | | | | | | |
| D | 1,03 | 39,3 | 25,200 | | 3,98 | **0,92** |
| E | 1,01 | 33,4 | 25,204 | | 3,03 | **0,89** |
| F | 1,10 | 38,3 | 25,031 (X) et 25,223 (LSX) | Aire Pic LSX > Aire Pic X | 3,75 | **0,93** |
| G | 1,12 | 37,4 | 25,003 (X) et 25,181 (LSX) | Aire Pic LSX > Aire Pic X | 3,65 | **0,98** |

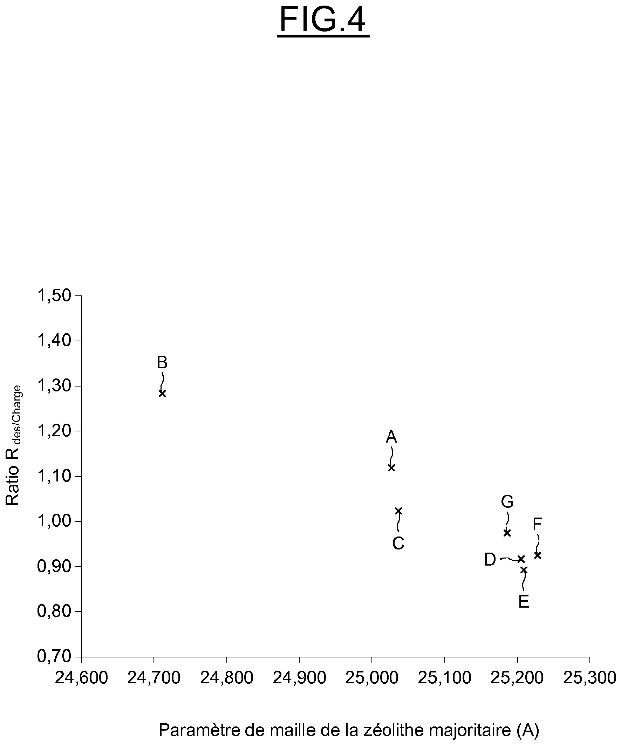

Le ratio volumique $R_{des/charge}$ des différents adsorbants A à G testés aux exemples 1 et 2 sont représentés graphiquement sur la figure 4 en fonction du paramètre de maille de la zéolithe majoritaire (LSX).

Exemple 3 (comparatif) :

Des tests de perçage/déperçage avec une charge contenant 10% en volume d'iso-octane utilisé comme traceur, 45% en volume de paraxylène PX et 45% en volume de métaxylène MX sont réalisés avec le solide D ayant un paramètre de maille conforme à l'invention, en utilisant un désorbant non conforme à l'invention, à savoir le paradiéthylbenzène (PDEB), et à une température de 175°C, couramment préconisée lorsque le PDEB est utilisé comme désorbant.
Le tableau 3 suivant indique la sélectivité PX/MX et le volume de désorbant injecté correspondant à Vdésorbant_équilibre, c'est-à-dire le volume nécessaire pour que la concentration en désorbant de l'effluent prélevé en sortie de colonne chromatographique devienne 100%.

Tableau 3 : Sélectivité PX/MX et volume de désorbant à l'équilibre en fonction du désorbant utilisé

| Adsorbant D | Paramètre de maille mesuré Å | $\alpha_{PX/MX}$ | Vdésorbant_équilibre (cm3) |
|---|---|---|---|
| PDEB à 175°C (comparatif) | 25,200 | 3,89 | 130,5 |
| TOL à 163°C (selon l'invention) | 25,200 | 4,15 | 105,6 |

Pour un même adsorbant comprenant une zéolithe LSX de paramètre de maille mesuré 25,200 Å et échangée au baryum (adsorbant D), le volume Vdésorbant_équilibre pour le cas du désorbant PDEB à 175°C est beaucoup plus grand que le volume Vdésorbant_équilibre pour le cas du désorbant Toluène à 163°C. Cela indique que la quantité de désorbant nécessaire pour désorber les composés de la charge adsorbés dans le volume microporeux est beaucoup plus faible lorsque l'on utilise le solvant toluène avec l'adsorbant D.

ANNEXE

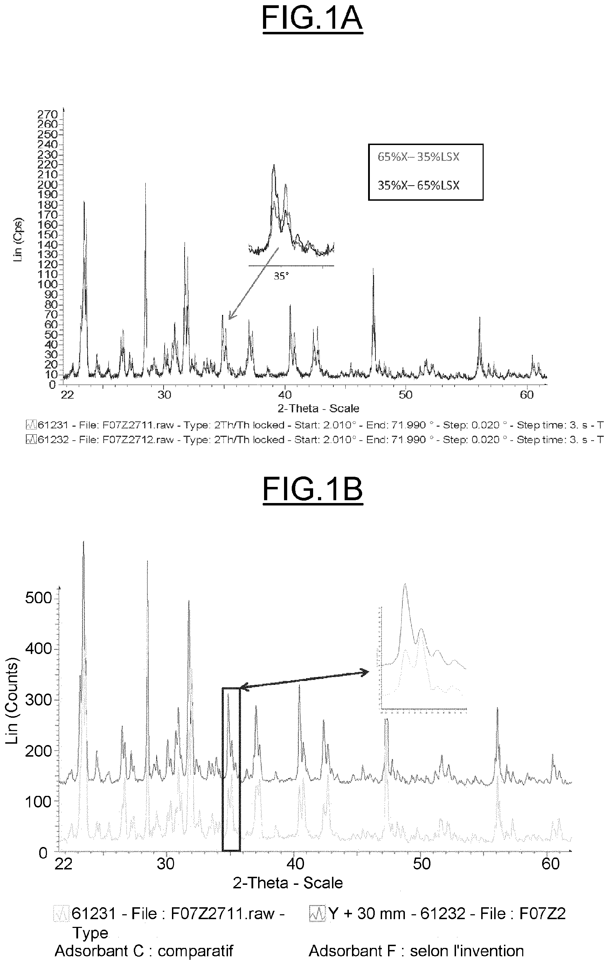

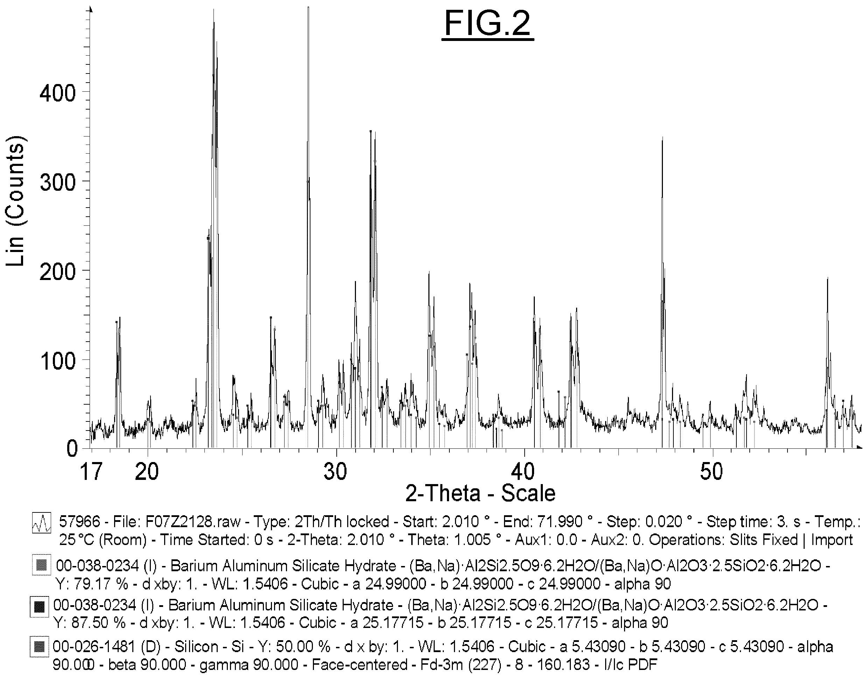

Tableau 4 : Tableau des positions et intensités des raies principales correspondant aux diffractogrammes des figures 1A, 1B et 2 des adsorbants C, F et G (valeurs du pic dédoublé à 35° en gras).

| Adsorbant C comparatif | | Adsorbant F selon l'invention | | Adsorbant G selon l'invention | |
|---|---|---|---|---|---|
| Angle | Intensité | **Angle** | Intensité | **Angle** | Intensité |
| 2-Theta ° | Count | 2-Theta ° | Count | 2-Theta ° | Count |
| 6.10 | 447 | 6.08 | 527 | 6.12 | 431 |
| 11.67 | 39 | 11.65 | 47 | 11.93 | 72 |

(suite)

| Adsorbant C comparatif | | Adsorbant F selon l'invention | | Adsorbant G selon l'invention | |
|---|---|---|---|---|---|
| Angle | Intensité | Angle | Intensité | Angle | Intensité |
| 12.22 | 370 | 12.16 | 377 | 12.23 | 418 |
| 14.04 | 146 | 14.04 | 220 | 14.08 | 191 |
| 14.12 | 155 | | | | |
| 15.43 | 57 | 15.32 | 43 | 15.34 | 53 |
| 18.29 | 84 | 18.27 | 90 | 18.30 | 93 |
| 18.41 | 140 | 18.40 | 92 | 18.44 | 136 |
| 23.14 | 169 | 23.12 | 234 | 23.18 | 236 |
| 23.35 | 371 | 23.38 | 503 | 23.44 | 462 |
| 23.56 | 429 | 23.56 | 269 | 23.60 | 420 |
| 24.46 | 58 | 24.45 | 84 | 24.50 | 81 |
| 26.45 | 94 | 26.43 | 135 | 26.49 | 109 |
| 26.63 | 146 | 26.64 | 101 | 26.67 | 126 |
| 27.16 | 61 | 27.14 | 85 | 27.21 | 58 |
| 27.34 | 70 | 27.36 | 55 | 27.41 | 63 |
| 29.20 | 79 | 29.18 | 75 | 29.24 | 77 |
| 30.05 | 75 | 30.05 | 107 | 30.11 | 89 |
| 30.29 | 100 | 30.28 | 68 | 30.34 | 86 |
| 30.70 | 84 | 30.69 | 123 | 30.75 | 104 |
| 30.91 | 165 | 30.90 | 170 | 30.96 | 173 |
| 31.13 | 114 | 31.13 | 75 | 31.17 | 107 |
| 31.72 | 240 | 31.71 | 383 | 31.78 | 322 |
| 31.96 | 362 | 31.95 | 218 | 32.01 | 325 |
| 32.56 | 82 | 32.57 | 56 | 32.64 | 70 |
| 33.56 | 75 | 33.56 | 59 | 33.60 | 65 |
| 33.86 | 58 | 33.90 | 72 | 33.95 | 72 |
| **34.83** | **124** | **34.82** | **199** | **34.89** | **175** |
| **35.10** | **153** | **35.11** | **105** | **35.15** | **151** |
| | | 36.85 | 76 | | |
| 37.02 | 129 | 37.01 | 175 | | |
| 37.11 | 133 | | | 37.10 | 162 |
| 37.29 | 143 | 37.29 | 97 | 37.34 | 138 |
| 40.43 | 128 | 40.43 | 216 | 40.50 | 157 |
| 40.75 | 146 | 40.75 | 101 | 40.80 | 131 |
| 42.36 | 92 | 42.37 | 146 | 42.43 | 139 |
| 42.70 | 151 | 42.70 | 97 | 42.75 | 152 |

**Revendications**

1. Procédé hybride de production de para-xylène de haute pureté à partir d'une charge de coupes d'isomères d'hydrocarbures aromatiques contenant 8 atomes de carbone, en phase liquide, comprenant :

   a) une étape de séparation par chromatographie liquide par adsorption à contre-courant simulé du para-xylène au moyen d'un adsorbant zéolithique et d'un solvant de désorption, ledit adsorbant comprenant :

      - au moins une zéolithe majoritaire de type faujasite de paramètre de maille « a » supérieur à 25,100 Å
      - du baryum telle que la teneur en oxyde de baryum BaO soit comprise entre 30% et 41% poids bornes incluses, par rapport au poids total de l'adsorbant,

   pour obtenir un flux de paraxylène purifié ;
   b) une étape de cristallisation du paraxylène à partir du flux purifié de paraxylène issu de l'étape de séparation, à une température comprise entre 0 et -25°C, suivie d'un lavage des cristaux avec un solvant de lavage, pour obtenir le paraxylène de haute pureté,
   le solvant de désorption dans l'étape de séparation et le solvant de lavage dans l'étape de cristallisation étant le toluène,
   l'adsorbant comprenant une zéolithe LSX ou un mélange de zéolithes de type faujasite, ce mélange étant une zéolithe X et une zéolithe LSX, la zéolithe LSX étant majoritaire.

2. Procédé de production de paraxylène selon la revendication 1 dans lequel la surface externe dudit adsorbant zéolithique, mesurée par adsorption d'azote, est inférieure à 100 m$^2$.g$^{-1}$.

3. Procédé de production de para-xylène selon la revendication 1 ou 2 dans lequel la teneur en oxyde de baryum est comprise entre 33 et 41% bornes incluses.

4. Procédé de production de para-xylène selon la revendication 3 dans lequel la teneur en oxyde de baryum est comprise entre 33 et 38% bornes incluses.

5. Procédé selon l'une des revendications précédentes dans lequel la température de l'étape de cristallisation est comprise entre -5°C et -15°C.

6. Procédé selon l'une des revendications précédentes dans lequel le taux de lavage des cristaux est compris entre 0,8 à 2 volumes de toluène par volume de cristaux de paraxylène.

7. Procédé selon l'une des revendications précédentes dans lequel le nombre de lits dans l'étape de séparation par adsorption est compris entre 4 et 24.

8. Procédé selon la revendication 7 dans lequel le nombre de lits dans l'étape de séparation par adsorption est compris entre 8 et 12.

9. Procédé selon l'une des revendications précédentes dans lequel l'étape de séparation par adsorption opère avec un nombre de lits compris entre 4 et 24, un nombre de zones au moins égal à 4, une température comprise entre 100°C et 250°C, une pression comprise entre la pression de bulle du toluène à la température du procédé et 3 MPa, un temps de cycle, correspondant au temps entre deux injections de désorbant sur un lit donné, compris entre 4 et 18 min, un rapport des débits de désorbant par rapport à la charge de 0,7 à 2,5, un taux de recyclage compris entre 2 et 12.

10. Procédé selon la revendication 9 dans lequel l'étape de séparation par adsorption opère à une température comprise entre 150°C et 180°C.

11. Procédé selon l'une des revendications précédentes dans lequel l'adsorbant est préparé selon les étapes suivantes :

    a) mélange des cristaux de zéolithe faujasite en poudre de granulométrie souhaitée, en présence d'eau avec au moins un liant à base d'une argile ou d'un mélange d'argiles, contenant au moins 80 % en poids d'argile zéolithisable et éventuellement une source de silice ;
    b) mise en forme du mélange obtenu en a) pour produire des agglomérés, puis séchage, éventuellement suivi d'une étape de tamisage et/ou de cyclonage ;

c) calcination des aggloméres obtenus en b) à une température allant préférentiellement de 500°C à 600°C ;

d) éventuelle zéolithisation du liant par mise en contact des aggloméres calcinés issus de l'étape c) avec une solution aqueuse basique alcaline suivie d'un lavage ;

e) échange ionique des aggloméres zéolithiques à base de zéolithe LSX, ou de X et LSX obtenus en c) ou en d) par des ions baryum, suivi d'un lavage et d'un séchage du produit ainsi traité ;

f) activation thermique des aggloméres échangés de l'étape e).

## Patentansprüche

1. Hybridverfahren zur Produktion von hochreinem para-Xylol aus einer Beschickung mit Isomerschnitten von aromatischen Kohlenwasserstoffen mit 8 Kohlenstoffatomen in flüssiger Phase, umfassend:

   a) einen Trennschritt durch flüssigchromatographische Trennung durch simulierte Gegenstromadsorption von para-Xylol unter Verwendung eines zeolithischen Adsorptionsmittels und eines Desorptionslösungsmittels, das Adsorptionsmittel umfassend:

   - mindestens einen mehrheitlichen Zeolith vom Typ Faujasit mit einem Maschenparameter "a" größer als 25,100 Å.
   - des Bariums derart, dass der Gehalt an Bariumoxid BaO zwischen 30 % und 41 % Gewichtsprozent einschließlich Grenzwerten liegt, bezogen auf das Gesamtgewicht des Adsorptionsmittels.

   um einen Strom von aufgereinigtem Paraxylen zu erlangen;

   b) einen Kristallisationsschritt des Paraxylens aus dem gereinigten Paraxylenstrom aus dem Trennschritt bei einer Temperatur zwischen 0 und -25 °C, gefolgt von einem Waschen der Kristalle mit einem Waschlösungsmittel, um hochreines Paraxylen zu erlangen,

   wobei das Desorptionslösungsmittel in dem Trennschritt und das Waschlösungsmittel in dem Kristallisationsschritt Toluol ist,

   das Adsorptionsmittel umfassend einen LSX-Zeolithen oder ein Gemisch aus Zeolithen vom Typ Faujasit, wobei dieses Gemisch ein X-Zeolith und ein LSX-Zeolith ist, wobei der LSX-Zeolith die Mehrheit ist.

2. Verfahren zur Produktion von Paraxylen nach Anspruch 1, wobei die Außenfläche des Zeolith-Adsorptionsmittels gemessen durch Stickstoffadsorption weniger als 100 m$^2$.g$^{-1}$ ist.

3. Verfahren zur Produktion von para-Xylol nach Anspruch 1 oder 2, wobei der Gehalt an Bariumoxid zwischen 33 und 41 % einschließlich der Grenzwerte liegt.

4. Verfahren zur Produktion von para-Xylol nach Anspruch 3, wobei der Gehalt an Bariumoxid zwischen 33 und 38 % einschließlich der Grenzwerte liegt.

5. Verfahren nach einem der vorherigen Ansprüche, wobei die Temperatur des Kristallisationsschritts zwischen -5 °C und -15 °C liegt.

6. Verfahren nach einem der vorherigen Ansprüche, wobei die Waschrate der Kristalle zwischen 0,8 und 2 Volumen Toluol pro Volumen Paraxylenkristalle liegt.

7. Verfahren nach einem der vorherigen Ansprüche, wobei die Anzahl der Betten in dem Trennschritt durch Adsorption zwischen 4 und 24 liegt.

8. Verfahren nach Anspruch 7, wobei die Anzahl der Betten in dem Trennschritt durch Adsorption zwischen 8 und 12 liegt.

9. Verfahren nach einem der vorherigen Ansprüche, wobei der Trennschritt durch Adsorption mit einer Anzahl von Betten zwischen 4 und 24, einer Anzahl von Zonen von mindestens 4, einer Temperatur zwischen 100 °C und 250 °C, einem Druck zwischen dem Blasendruck des Toluols bei der Prozesstemperatur und 3 MPa, einer Zykluszeit, die der Zeit zwischen zwei Injektionen von Desorptionsmittel auf ein gegebenes Bett entspricht, zwischen 4 und 18 Min, einem Verhältnis der Desorptionsmitteldurchsätze zur Charge von 0,7 bis 2,5, einer Recyclingrate zwischen 2 und 12 betrieben wird.

10. Verfahren nach Anspruch 9, wobei der Trennschritt durch Adsorption bei einer Temperatur zwischen 150 °C und 180 °C betrieben wird.

11. Verfahren nach einem der vorherigen Ansprüche, wobei das Adsorptionsmittel gemäß den folgenden Schritten präpariert wird:

a) Mischen der pulverförmigen Faujasit-Zeolithkristalle mit der gewünschten Korngröße in Anwesenheit von Wasser mit mindestens einem Bindemittel auf Basis eines Tons oder eines Gemischs von Ton, das mindestens 80 Gewichtsprozent zeolithisierbaren Ton und optional eine Siliciumdioxidquelle enthält;
b) Formen des in a) erlangten Gemischs zur Produktion von Agglomeraten, dann Trocknen, eventuell gefolgt von einem Sieb- und/oder Zyklonierungsschritt;
c) Kalzinieren der in b) erlangten Agglomerate bei einer Temperatur, die vorzugsweise von 500 °C bis 600 °C reicht;
d) optional Zeolithisieren des Bindemittels durch Inkontaktbringen der kalzinierten Agglomerate aus Schritt c) mit einer wässrigen basischen Alkalilösung und anschließendem Waschen;
e) Austauschen von Ionen der Zeolithagglomerate auf Basis von Zeolith LSX oder von X und LSX, die in c) oder d) erlangt wurden, mit Bariumionen, gefolgt von Waschen und Trocknen des so behandelten Produkts;
f) thermisches Aktivieren der ausgetauschten Agglomerate aus Schritt e).

**Claims**

1. Hybrid process for producing high-purity para-xylene from a feedstock of aromatic hydrocarbon isomer fractions having 8 carbon atoms, in a liquid phase, comprising:

a) a liquid chromatography separation step via simulated countercurrent adsorption of para-xylene using a zeolitic adsorbent and a desorption solvent, said adsorbent comprising:

- at least one majority zeolite of faujasite type having a lattice parameter «a» higher than 25.100 Å,
- barium such that the content of barium oxide BaO is comprised between 30 % and 41 % by weight, limits included, relative to the total weight of the adsorbent,

to obtain a stream of purified para-xylene;
b) a crystallization step of the para-xylene from the purified stream of para-xylene obtained at the separation step, at a temperature between 0 and -25 °C, followed by washing of the crystals with a washing solvent, to obtain high-purity para-xylene,
wherein the desorption solvent at the separation step and the washing solvent at the crystallization step is toluene,
wherein the adsorbent comprises an LSX zeolite or a mixture of zeolites of faujasite type, this mixture being a X-type zeolite and LSX zeolite, the LSX zeolite being in majority.

2. The process for producing para-xylene according to claim 2, wherein the outer surface area of said zeolitic adsorbent, measured by nitrogen adsorption, is less than 100 $m^2.g^{-1}$.

3. The process for producing para-xylene according to claims 1 or 2, wherein the content of barium oxide is between 33 and 41 %, limits included.

4. The process for producing para-xylene according to claim 3, wherein the content of barium oxide is between 33 and 38 %, limits included.

5. The process according to any of the previous claims, wherein the temperature of the crystallization step is between -5 °C and -15 °C.

6. The process according to any of the previous claims, wherein the wash ratio of the crystals is between 0.8 and 2 volumes of toluene per volume of para-xylene crystals.

7. The process according to any of the previous claims, wherein the number of beds at the adsorption separation step is between 4 and 24.

8. The process according to claim 7, wherein the number of beds at the adsorption separation step is between 8 and 12.

9. The process according to any of the previous claims, wherein the adsorption separation step is conducted with a number of beds comprised between 4 and 24, a number of zones of at least 4, a temperature between 100 °C and 250 °C, a pressure comprised between toluene bubble point pressure at processing temperature and 3 MPa, a cycle time corresponding to the time interval between two injections of desorbent into a given bed of between 4 and 18 min, a ratio of desorbent flow rate to feed flow rate of 0.7 to 2.5, and a recycle ratio comprised between 2 and 12.

10. The process according to claim 9, wherein the adsorption separation step is conducted at a temperature comprised between 150 °C and 180 °C.

11. The process according to any of the previous claims, wherein the adsorbent is prepared according to the following steps:

> a) mixing the faujasite zeolite crystals in powder form of desired particle size, in the presence of water, with at least one binder containing a clay or a mixture of clays comprising at least 80 % by weight of zeolitizable clay and optionally a silica source;
> b) forming the mixture obtained at a) to produce aggregates, followed by drying and optionally a screening and/or cyclonic separation step;
> c) calcining the aggregates obtained at b) at a temperature preferably in the range of 500 °C to 600 °C;
> d) optional zeolitization of the binder by contacting the calcined aggregates obtained at step c) with an alkaline basic aqueous solution followed by washing;
> e) ion exchange of the zeolitic aggregates containing LSX zeolite or X and LSX zeolite obtained at c) or d) with barium ions, followed by washing and drying of the product thus treated;
> f) heat activation of the aggregates exchanged at step e).

# FIG.1A

61231 - File: F07Z2711.raw - Type: 2Th/Th locked - Start: 2.010 ° - End: 71.990 ° - Step: 0.020 ° - Step time: 3. s - T
61232 - File: F07Z2712.raw - Type: 2Th/Th locked - Start: 2.010 ° - End: 71.990 ° - Step: 0.020 ° - Step time: 3. s - T

# FIG.1B

61231 - File : F07Z2711.raw - Type          Y + 30 mm - 61232 - File : F07Z2

Adsorbant C : comparatif          Adsorbant F : selon l'invention

## FIG.2

57966 - File: F07Z2128.raw - Type: 2Th/Th locked - Start: 2.010 ° - End: 71.990 ° - Step: 0.020 ° - Step time: 3. s - Temp.: 25°C (Room) - Time Started: 0 s - 2-Theta: 2.010 ° - Theta: 1.005 ° - Aux1: 0.0 - Aux2: 0. Operations: Slits Fixed | Import

00-038-0234 (I) - Barium Aluminum Silicate Hydrate - (Ba,Na)·Al2Si2.5O9·6.2H2O/(Ba,Na)O·Al2O3·2.5SiO2·6.2H2O - Y: 79.17 % - d xby: 1. - WL: 1.5406 - Cubic - a 24.99000 - b 24.99000 - c 24.99000 - alpha 90

00-038-0234 (I) - Barium Aluminum Silicate Hydrate - (Ba,Na)·Al2Si2.5O9·6.2H2O/(Ba,Na)O·Al2O3·2.5SiO2·6.2H2O - Y: 87.50 % - d xby: 1. - WL: 1.5406 - Cubic - a 25.17715 - b 25.17715 - c 25.17715 - alpha 90

00-026-1481 (D) - Silicon - Si - Y: 50.00 % - d x by: 1. - WL: 1.5406 - Cubic - a 5.43090 - b 5.43090 - c 5.43090 - alpha 90.000 - beta 90.000 - gamma 90.000 - Face-centered - Fd-3m (227) - 8 - 160.183 - I/Ic PDF

EP 3 679 004 B1

# FIG.3A

◇ %Vol iC8   ✳ %Vol PX   ○ %Vol MX   ▵ %Vol Toluène

Test de perçage :
injection de la charge
10% traceur iC8
45% PX
45% MX

Fraction
volumique

VR_MX

Vnon_sélectif    VR_PX

Vcharge_équilibre

Volume élué expérimental cm³

# FIG.3B

◇ %Vol iC8   ✳ %Vol PX   ○ %Vol MX   ▵ %Vol Toluène

Test de déperçage :
injection du désorbant

Fraction
volumique

VR_MX

Vnon_sélectif    VR_PX

Vdésorbant_équilibre

Volume élué expérimental (cc)

# FIG.4

Paramètre de maille de la zéolithe majoritaire (A)

**EP 3 679 004 B1**

## RÉFÉRENCES CITÉES DANS LA DESCRIPTION

**Documents brevets cités dans la description**

- FR 2681066
- US 2985589 A
- US 4402832 A
- US 4498991 A
- US 3177265 A
- US 3467724 A
- CN 103508837
- FR 2693187
- EP 0531191 A
- US 3558730 A
- US 3558732 A
- US 3663638 A
- US 3878127 A
- EP 2237878 A
- EP 2237877 A
- WO 2016075281 A
- FR 2903978
- FR 2925367
- FR 2925366
- FR 3028430